# EUROPEAN PATENT APPLICATION

(11) **EP 2 615 181 A1**
(43) Date of publication of application: **17.07.2013**
(21) Application number: 12305031.2
(22) Date of filing: 10.01.2012
(51) Int. Cl.: C12Q 1/68, A61K 39/00

(54) **Cyclon expression for the identification and control of cancer cells**

(71) Applicant: Universite Joseph Fourier - Grenoble 1, 38400 St. Martin d'Heres (FR); Centre Hospitalier Universitaire Grenoble, 38700 La Tronche (FR); Centre National de la Recherche Scientifique (C.N.R.S.), 75016 Paris (FR); Commissariat à l'Énergie Atomique et aux Énergies Alternatives, 75015 Paris (FR)
(72) Inventor: Callanan Mary, 38706 LA TRONCHE (FR); Emadali, Anouk, 38000 GRENOBLE (FR); Ferro, Myriam, 38120 FONTANIL-CORNILLON (FR); Khochbin, Saadi, 38240 MEYLAN (FR); Pison-Rousseaux, Sophie, 38410 ST MARTIN D'URIAGE (FR)
(74) Representative: Regimbeau

(57) **Abstract**

The present invention concerns a method for the identification of the presence or absence of cancer cells in a biological sample, more particularly a method for the identification of the susceptibility or resistance to a treatment with CD20 agonists of cancer cells in a biological sample, said methods comprising determining the level of expression of the gene Cyclon in said cells and comparing said level of expression to the level of expression in a non-cancer cell, wherein a level of expression higher than the level of expression in a non-cancer cell is an indication of the presence of a cancer cell, more particularly an indication of cancer cells with a resistance to treatment with antagonists.
The invention also concerns kits for the determination of a level of expression of the Cyclon gene in a biological sample, a method for the identification of Cyclon expression antagonists, as well as methods for treating patients in need thereof with CD20 agonists when the patients are identified to be susceptible to such a treatment and/or treating patients in need thereof with a combination of a Cyclon inhibitor and a CD20 agonists.

SEQUENCE LISTING

<110>  CENTRE HOSPITALIER UNIVERSITAIRE GRENOBLE
       CENTRE NATIONAL DE LA RECHERCHE SCIENTIFIQUE (CNRS)
       UNIVERSITE JOSEPH FOURIER - GRENOBLE 1
       COMMISSARIAT A L'ENERGIE ATOMIQUE ET AUX ENERGIES
       ALTERNATIVES

<120>  Cyclon expression for the identification and control of cancer
       cells

<130>  69735D30579

<160>  8

<170>  PatentIn version 3.5

<210>  1
<211>  77
<212>  DNA
<213>  Artificial Sequence

<220>
<223>  Oligonucleotide Cyclonsh2F

<400>  1
cgtgtaccgg tccgtccaag aagttgaata aagctcgagc tttattcaac ttcttggacg      60

tttttgaatt ccgtcgt                                                     77


<210>  2
<211>  77
<212>  DNA
<213>  Artificial Sequence

<220>
<223>  Oligonucleotide Cyclonsh2R

<400>  2
acgacggaat tcaaaaacgt ccaagaagtt gaataaagct cgagctttat tcaacttctt      60

ggacggaccg gtacacg                                                     77


<210>  3
<211>  77
<212>  DNA
<213>  Artificial Sequence

<220>
<223>  Oligonucleotide Cyclonsh3F

<400>  3
cgtgtaccgg tccccagatg cttcaggaca agcctcgagg cttgtcctga agcatctggg      60

tttttgaatt ccgtcgt                                                     77


<210>  4
<211>  77
<212>  DNA
<213>  Artificial Sequence

<220>

<223>  Oligonucleotide Cyclonsh3R

<400>  4
acgacggaat tcaaaaaccc agatgcttca ggacaagcct cgaggcttgt cctgaagcat      60

ctggggaccg gtacacg                                                     77


<210>  5
<211>  77
<212>  DNA
<213>  Artificial Sequence

<220>
<223>  Oligonucleotide CtrlshF

<400>  5
cgtgtaccgg tccctaaggt taagtcgccc tcgctcgagc gagggcgact taaccttagg      60

tttttgaatt ccgtcgt                                                     77


<210>  6
<211>  77
<212>  DNA
<213>  Artificial Sequence

<220>
<223>  Oligonucleotide CtrlshR

<400>  6
acgacggaat tcaaaaacct aaggttaagt cgccctcgct cgagcgaggg cgacttaacc      60

ttagggaccg gtacacg                                                     77


<210>  7
<211>  20
<212>  DNA
<213>  Artificial Sequence

<220>
<223>  Oligonucleotide CyclonF

<400>  7
agcgaaaagg ttcttcatcc                                                  20


<210>  8
<211>  20
<212>  DNA
<213>  Artificial Sequence

<220>
<223>  Oligonucleotide CyclonR

<400>  8
ttcctctcct gtcgttcctt                                                  20

## Description

The present invention concerns a method for the identification of the presence or absence of cancer cells in a biological sample, more particularly a method for the identification of the susceptibility or resistance to a treatment with CD20 agonists of cancer cells in a biological sample, said methods comprising determining the level of expression of the gene Cyclon in said cells and comparing said level of expression to the level of expression in a non-cancer cell, wherein a level of expression higher than the level of expression in a non-cancer cell is an indication of the presence of a cancer cell, more particularly an indication of cancer cells with a resistance to treatment with antagonists.

The invention also concerns kits for the determination of a level of expression of the Cyclon gene in a biological sample, a method for the identification of Cyclon expression antagonists, as well as methods for treating patients in need thereof with CD20 agonists when the patients are identified to be susceptible to such a treatment and/or treating patients in need thereof with a combination of a Cyclon inhibitor and a CD20 agonists.

### BACKGROUND OF THE INVENTION

Monoclonal antibodies represent an effective modality for cancer treatment. Their effect on cancer cells is thought to function by several mechanisms including antibody dependant cellular cytotoxicity (ADCC), stimulation of complement-dependant cytotoxicity (CDC) and/or direct induction of apoptosis (Cheson and Leonard, 2008). However, monoclonal antibodies alone are not curative and must be used in association with chemotherapeutic agents to be effective treatments for human cancer.

Rituximab is a chimeric IgG1 monoclonal antibody that specifically targets the CD20 surface antigen expressed on normal and neoplastic lymphoid cells. Based on its safety and effectiveness in clinical trials, Rituximab was the first monoclonal antibody approved by the FDA for cancer treatment in 1997 for its use in B cell non-Hodgkin lymphoma resistant to other chemotherapy regimens. Rituximab, in combination with CHOP chemotherapy, is now the standard therapy in the initial treatment of B cell non-Hodgkin lymphomas and significantly improves long-term survival.

Anti-CD20-directed therapeutic approaches are also increasingly being used in the maintenance or pre-emptive therapy setting in several lymphoma subtypes. In the latter setting, anti-CD20 antibodies alone are administered to patients who are in complete clinical remission in order to maintain responses over time and to control residual disease (lymphoma stem cells).

Despite its demonstrated clinical effectiveness, persistent lack of clarity regarding the precise mode of action for anti-CD20 monoclonal antibodies is reflected by the variety of approaches undertaken to improve the potency of these reagents. These strategies essentially involve the development of more effective antibodies derived from Rituximab as well as innovative combined treatment modalities designed to optimize its mechanism of action.

Unresponsiveness or resistance of a substantial proportion of B-cell lymphomas and various responses according to tumour burden and subtype implies a need for new strategies to increase potency and broaden the spectrum of anti-CD20 therapy. Moreover, new therapeutic strategies might avoid the toxic effect of chemotherapy, improve the outcomes when combined with chemotherapy and provide options for patients with refractory diseases.

### SUMMARY OF THE INVENTION

The present inventors have uncovered the potential of the gene Cyclon to be used as a diagnostic marker, a prognosis marker that more specifically predict CD20 agonists' efficacy and as a therapeutic target to increase cancer cell sensitivity to CD20 agonists.

Accordingly, the present invention concerns a method for the identification of the presence or absence of cancer cells in a biological sample comprising cells obtained from a patient, said method comprising measuring the level of expression of the gene Cyclon in said cells in the biological sample and comparing the measured level of expression with a standard level of expression in a non-cancer cell, wherein a level of expression higher than the standard level of expression is an indication of the presence of a cancer cell.

The invention also concerns a method for the identification of the susceptibility or resistance to a treatment with CD20 agonists of cancer cells in a biological sample comprising cancer cells obtained from a patient, said method comprising measuring the level of expression of the gene Cyclon in said cancer cells in the biological sample and comparing the measured level of expression with a standard level of expression in a non-cancer cell, wherein a level of expression higher than the standard level of expression is an indication of a resistance to treatment with a CD20 agonists.

The invention also concerns a kit for its use in the methods of the invention comprising means for determining the level of expression of Cyclon in cells comprised in a biological sample.

The invention also concerns a CD20 agonist for its use in the treatment of a cancer susceptible to be treated with CD20 agonists, wherein the treatment comprises identifying patients comprising cancer cells susceptible to a treatment with CD20 agonists with the method described above and administering the CD20 agonists to said patients.

The invention also concerns a combination product comprising a CD20 agonist and a Cyclon inhibitor, for their use combined or separately, simultaneously or in sequence, in the treatment of cancers susceptible to be treated with CD20 agonists, particularly for patients susceptible to be treated with CD20 agonists previously identified with a method of the invention.

The invention also concerns a method for the identification of compounds susceptible to inhibit the expression of Cyclon in a cancer cell overexpressing Cyclon, comprising contacting a sample comprising said cancer cells overexpressing Cyclon with one or more compounds to be tested, measuring the expression level of Cyclon, and comparing the measured expression level with the expression level of the cancer cell over expressing Cyclon, and selecting the compounds inducing a decrease in said Cyclone expression level.

Further, the present invention also concerns a method for diagnosing the presence or absence of cancer cells in a biological sample comprising cells obtained from a patient, said method comprising:
i. measuring the level of expression of the gene Cyclon in said cells in the biological sample; and
ii. comparing the measured level of expression with a standard level of expression in a non-cancer cell,
wherein a level of expression higher than the standard level of expression is an indication of the presence of a cancer cell.

The invention also concerns a method for predicting the prognosis of an individual diagnosed with a cancer susceptible to treatment with CD20 agonists, said method comprising:
i. measuring the level of expression of the gene Cyclon in said cancer cells in the biological sample; and
ii. comparing the measured level of expression with a standard level of expression in a non-cancer cell,
wherein a level of expression higher than the standard level of expression is an indication of a resistance to treatment with a CD20 agonists.

The invention also concerns a method of treating a cancer susceptible to be treated with CD20 agonists, wherein the treatment comprises administering to an individual a CD20 agonist and a Cyclon inhibitor. The CD20 agonists and Cyclon inhibitor may be a combination product, used combined or separately, simultaneously or in sequence. In one embodiment, the method further comprises the step of identifying patients comprising cancer cells susceptible to a treatment with CD20 agonists with the method described above.

Accordingly, the invention also concerns a method of increasing the sensitivity of cells to a CD20 agonists, said method comprising decreasing the level of expression of Cyclon.

### DETAILLED DESCRIPTION OF THE INVENTION

Except as mentioned otherwise, the terms and expressions below are defined as follows.

"Cyclon" or "gene Cyclon" is the gene identified in Unigene as Hs.4253. Cyclon has been identified as a cytokine-inducible gene expressed in response to IL3 (Hoshino and Fujii, 2007). Cyclon has also been identified as a Fas-dependant regulator of immune homeostasis in T cells (Saint Fleur et al., 2009), but no other structural or functional information is available about this gene product. We first identified this gene product in relation to cancer. "Measuring" or "determining" the level of expression of the gene Cyclon is made by known techniques such as quantitative PCR-based techniques with primers specifically targeting the gene Cyclon or with antibodies specifically targeting the gene product of Cyclon in a biological sample. Such methods are well known in the art and include RNA preparation followed by quantitative PCR, flow cytometry and immunohistochemistry. In the context of the present invention "measuring" and "determining" are used interchangeably with no distinct meaning.

"A biological sample comprising cells obtained from a patient" is obtained by usual means of sampling cells from biological tissues, in a solid or liquid form. Said cells are treated and conserved in manners known to the skilled person to allow reliable determination of the expression level of Cyclon. Methods for obtaining a biological sample comprising cells are well known in the art and include invasive methods such as biopsy or surgery.

"A biological sample comprising cancer cells obtained from a patient" is a sample obtained from a patient already diagnosed with cancer. In such case, the method of the invention may be performed on biological samples obtained from surgery intended to remove cancer cells. It can also be performed on biological samples maintained and stored in a cell bank or biological resource centre, wherein said cells are known to belong to a patient having cancer.

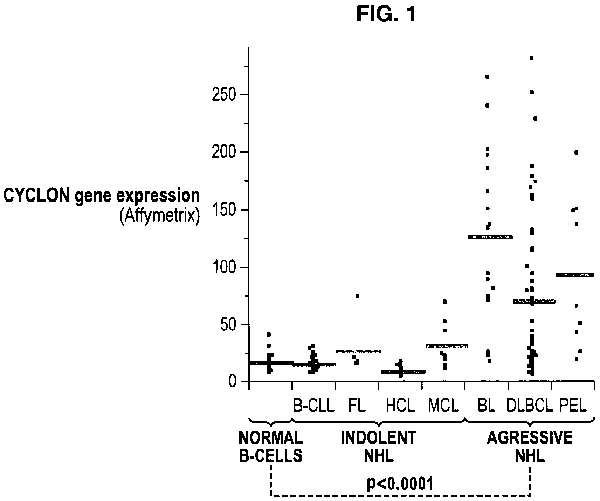

"Standard level of expression" is the level of expression measured in cells known to be non-cancer cells (Figure1). Cyclon is known to have a low level of expression in non-cancer cells, below a threshold of expression defined by M+2*sd (M= mean value of expression in a set of normal adult somatic tissues; sd = standard deviation

characterizing the distribution of the same values; the adult somatic tissues should preferably comprise at least 30 samples from 15 different origins). Therefore, measuring a level of expression of Cyclon in a sample of cells above an upper standard level of expression M+2*sd, means the cells are cancer cells.

However, some cancer cells may have a lower level of expression of Cyclon, comparable to the level of expression of non-cancer cells, below the upper standard level of expression of M+2*sd. For such cells, this is an indication that said cancer cells are likely to be susceptible to a treatment with CD20 agonists. On the other hand, finding a level of expression above the said upper standard of expression is an indication that said cancer cells are likely to be resistant to a treatment with CD20 agonists.

A method for the identification of the presence or absence of cancer cells, or a method for the identification of the susceptibility or resistance to a treatment with CD20 agonists of cancer cells, in a biological sample comprising cells or cancer cells obtained from a patient is also a method for determining whether an individual (a patient) has cancer or whether the individual has a cancer resistant or susceptible to a treatment with CD20 agonists, i.e. a method of diagnosis and/or prognosis. Said methods indeed include a first step of obtaining a biological sample comprising cells as defined above.

Cancer cells of cancers susceptible to be treated with CD20 agonists are selected among lymphomas, colon, prostate, stomach and bladder cancers. The method of the invention is particularly appropriate in case of suspicion of the presence of one of these cancers.

The method of the invention is particularly appropriate to identify cancers susceptible to be treated with CD20 agonists particularly CD20-positive, B-cell non-Hodgkin's lymphoma, such as Diffuse Large B cell Lymphoma and Follicular Lymphoma (non exhaustive list).

The method of the invention is particularly suitable to identify the individual having an aggressive non-Hodgkin's lymphoma being susceptible to be treated with a CD20 agonist.

"CD20 agonists" are known in the art, some being already marketed products being used in the treatment of cancers in patients in need thereof. CD20 agonist comprises monoclonal antibodies, polyclonal antibodies and antibodies fragments. These antagonists are particularly selected among the group consisting of anti-CD20 monoclonal antibodies. Known anti-CD20 antibodies, particularly humanized monoclonal antibodies, are Rituximab (sold under the trade names Rituxan and MabThera), or second/third generation antibodies such as Ofatumumab, Ocrelizumab, PRO131921, Veltuzumab, AME-133, Tositumomab and GA-101 (Robak and Robak, 2011).

Preferred CD20 agonists are anti-CD20 monoclonal antibodies, such as Rituximab and other monoclonal antibodies having a similar biological activity, particularly biosimilars.

"Kits" for use in the methods of the invention comprising means for determining the level of expression of Cyclon in cells comprised in a biological sample are also known in the art. The means depends on the method of detection chosen to determine the level of expression of Cyclon, by PCR or antibodies. The kit may further comprise additional reagent to perform the methods of determination of a level of expression of a gene, also known to the skilled persons. It may also comprise vials to perform the reactions necessary for determining the level of expression of a gene as well as operating instructions to perform the methods of the invention, said instructions being either printed on a paper and/or available on an electronic format, on a specific support for electronic data (cd-rom, USB drive, etc.) or available on line on a specific web site.

A high level of expression of Cyclon - above the upper standard level as defined above - may be associated with a high level of expression of other biological factors, such as IL1A, KIF14, NUMA1 and CARM1 (non exhaustive list) or a low level of expression of other biological factors IKZF3, MEF2C, INCENP and MED18 (non exhaustive list). These factors linked to the expression of Cyclon are additional markers confirming the high level of expression of Cyclon (see Table 1 for top list of Cyclon regulated genes).

The methods of the invention may also comprise, in addition to determining the level of expression of Cyclon, determining the absence or presence of said additional markers to confirm the presence or absence of cancer cells and/or the susceptibility or resistance to cancer cells to a treatment with CD20 agonists.

Therefore, the kit of the invention may also comprise, in addition to the means necessary to measure the level of expression of Cyclon, means necessary to determine the presence of said additional markers.

The invention therefore comprises a method for treating a patient in need thereof, said patient having a cancer, said method comprising determining whether said patient is susceptible to be treated with a CD20 agonist by measuring the level of expression of Cyclon in a sample of cancer cells obtained with said patient, and administering a CD20 agonist to said patient when the level of expression of Cyclon in said cancer cells is below the upper standard level of expression of Cyclon.

In selecting a population of patients having a low level of expression of Cyclon, it is possible to improve the efficacy of a treatment with CD20 agonists as defined above.

The inventors also found that inhibiting expression of Cyclon in cancer cells otherwise resistant to a treatment with CD20 agonists makes said cells susceptible to a treatment with a CD20 agonist. Accordingly, the invention comprises a method of increasing the sensitivity of cells to a CD20 agonist by decreasing the expression of Cyclon.

Therefore, the invention also comprises a method for treating a patient in need thereof, said patient having cancer, comprising combining administering a Cyclon inhibitor to said patient and administering a CD20 agonist. It also comprises a combination product comprising a CD20 agonist and a Cyclon inhibitor, for their use combined or separately, simultaneously of in sequence, in the treatment of cancers susceptible to be treated with CD20 agonists.

The combined administration may be performed simultaneously, both products being present combined in the same formulation or individually in two formulations being administered simultaneously. The combined administration may also be done in sequence, administering first the Cyclon inhibitor to allow reduction of the resistance of the cancer cells to the CD20 agonist and then administering the said CD20 agonist.

The skilled person shall determine the best amount of each of the drugs (Cyclon inhibitor as defined below and CD20 agonists as defined above) as well as the best sequence of administration, depending of the specifics of each patient, particularly based on the level of expression of Cyclon in said cancer cells measured according to the method of the invention.

"Cyclon inhibitors" are known in the art or can be readily identified with the method of the invention. In one embodiment, the level of expression of Cyclon may be controlled upstream by several biological factors, such as CD40, the activation of which increases the expression of Cyclon. Other factors are known to favour the expression of Cyclon such as cytokines. Antagonists of said factors are known in the art, including CD40 blocking antibody. In a preferred embodiment, such antagonists are CD40 antagonists, particularly selected among CD40 monoclonal antibodies (HCD122) or pathway inhibitory molecules (PG102).

In a preferred embodiment, the combination product or the method of treatment with combined administration comprises an anti-CD40 monoclonal antibody, such as HCD1222, or any antibody having similar biological activity, particularly any biosimilar, as Cyclon inhibitor, and an anti-CD20 monoclonal antibody, such as Rituximab, or any antibody having similar biological activity, particularly any biosimilar, as CD20 agonist.

Other "small" molecules, such as Imatinib, sold under the name Glivec, have also been shown to reduce expression level. Combined treatment or combination product comprising Imatinib as Cyclon inhibitor and Rituximab as CD20 agonist are also preferred embodiments of the invention.

The methods of treatment and combination product of the invention are particularly suitable for patients being affected by lymphoma, particularly non-Hodgkin's lymphoma, more particularly aggressive non-Hodgkin's lymphoma, such as Diffuse Large B Cell Lymphoma. Other cancers susceptible to be treated with the methods and combination product of the invention are selected among colon, prostate, stomach and bladder cancers.

It is understood that the method of treatment and combination product of the invention are generally not used with patients showing complex cancers such as multiple coexisting cancers. Particularly, combined treatment of complex cancers with rituximab and imatinib, selected among chronic myeloid leukaemia and relapsed non-Hodgkin lymphoma (Breccia et al., 2008)., chronic myeloid leukaemia and hairy cell leukaemia (Orciuolo et al., 2006) and simultaneous relapse of MALT lymphoma and a gastrointestinal stromal tumour (Bompas et al., 2004) are not part of the present invention.

The invention also concerns a method for the identification of compounds susceptible to inhibit the activity of Cyclon or expression of Cyclon in a cancer cell overexpressing Cyclon. Said method may be performed on compounds alone as well as on a library of compounds. The person skilled in the art will be able to perform a test usually known to perform such methods for determining whether compounds are inhibiting the expression of a known gene. Such methods comprise screening of chemical library or using public transcriptomic and/or published data to identify compounds known to reduce Cyclon expression.

### BRIEF DESCRIPTION OF THE FIGURES

**Figure 1****:** Cyclon is significantly overexpressed in aggressive lymphoma (BL: Burkitt Lymphoma, DLBCL: Diffuse Large B cells Lymphoma, PEL : Primary Effusion Lymphoma) compared to normal B cells or indolent lymphoma (B-CLL : B chronic lymphocytic leukaemia, FL : Follicular lymphoma, MCL: Mantle Cell Lymphoma, HCL: Hairy cell leukaemia). Affymetrix expression data retrieved from Basso study (Basso et al., 2005).

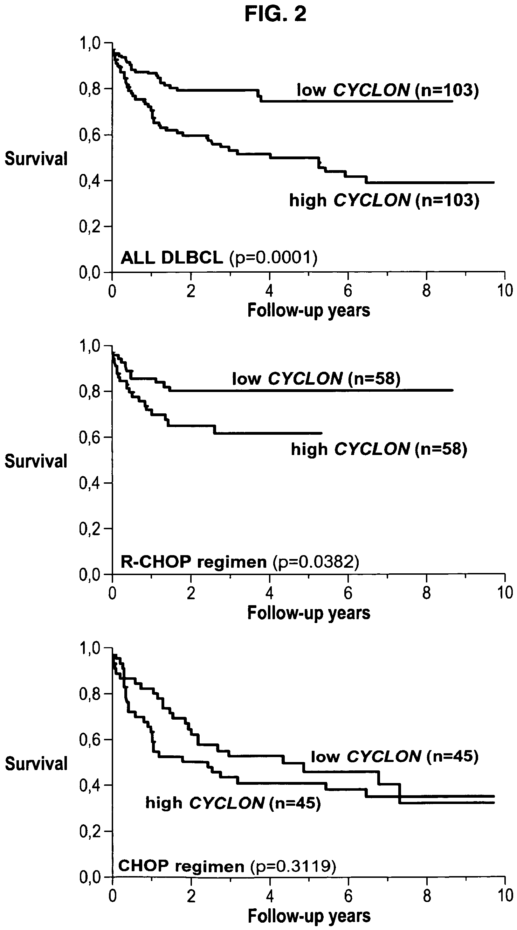

**Figure 2****:** Kaplan-Meier cumulative survival curves drawn from Lenz data, p values from Mantel-Cox test (n=414)

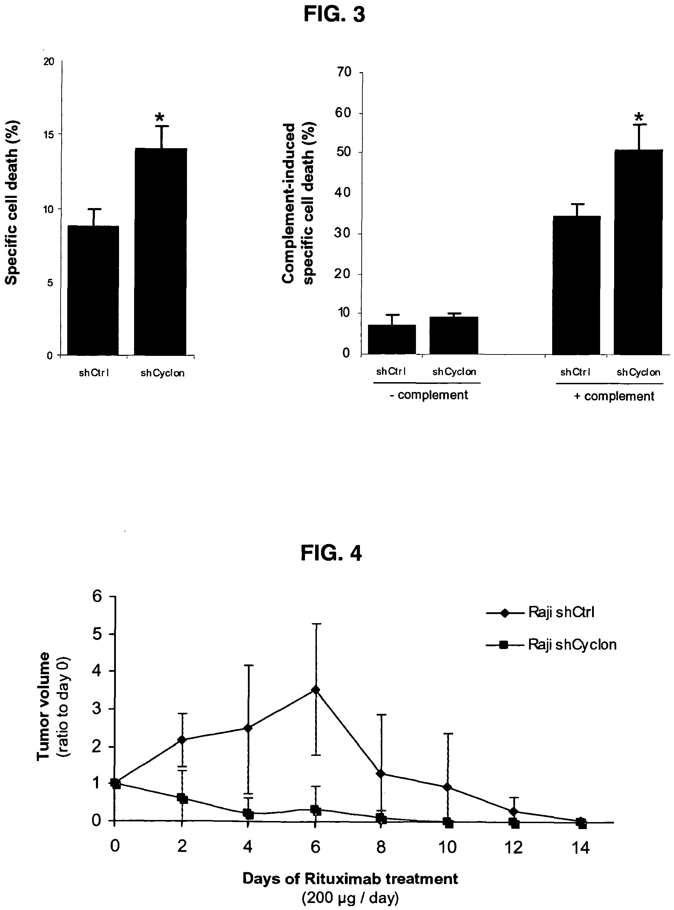

**Figure 3****:** Direct apoptosis - specific cell death of Raji cells (either control - sh Ctrl or silenced for Cyclon expression - sh Cyclon) when treated for 24 h with 10 µg/ml Rituximab (left panel). CDC specific cell death of Raji cells (either control - sh Ctrl or silenced for Cyclon expression - sh Cyclon) when treated for 1 h with 1 µg/ml Rituximab (right panel). *p<0.02 (derived from a Wilcoxon test)
**Figure 4****:** Tumour volume was measured with the average volume shown for control tumours derived from Raji shCtrl line or Cyclon knock-down tumours (derived from Raji shCyclon line). p<0.001 (derived from a Wilcoxon test).

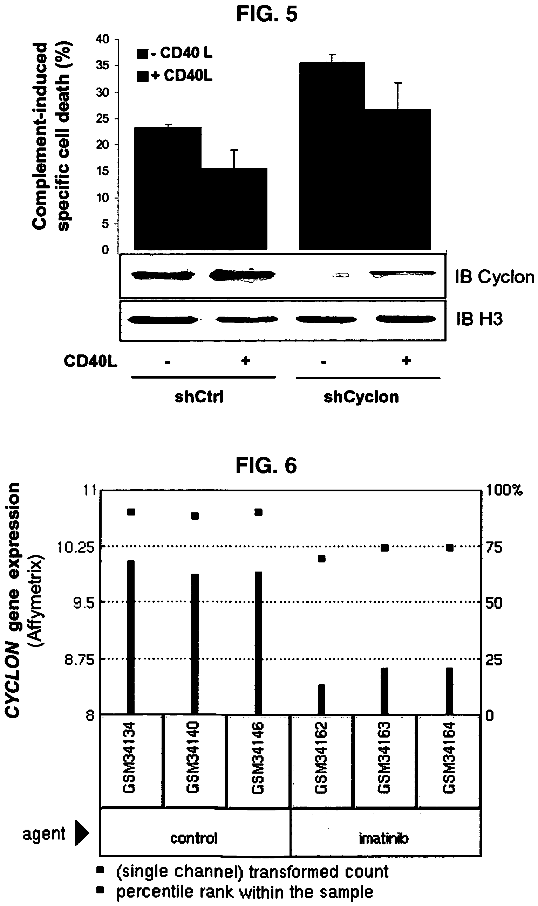

**Figure 5****:** CDC specific cell death of Raji cells (either control - sh Ctrl or silenced for Cyclon expression - sh Cyclon) when treated for 48 h with 0.1 mg/ml CD40 ligand and then treated for 1 h with 1 µg/ml Rituximab (upper panel) and reciprocal western blot analysis of Cyclon expression level (bottom panel).
**Figure 6****:** Transcriptomic gene expression data (GDS3043) showing a down-regulation of Cyclon in K562 leukemic cells treated with Imatinib.

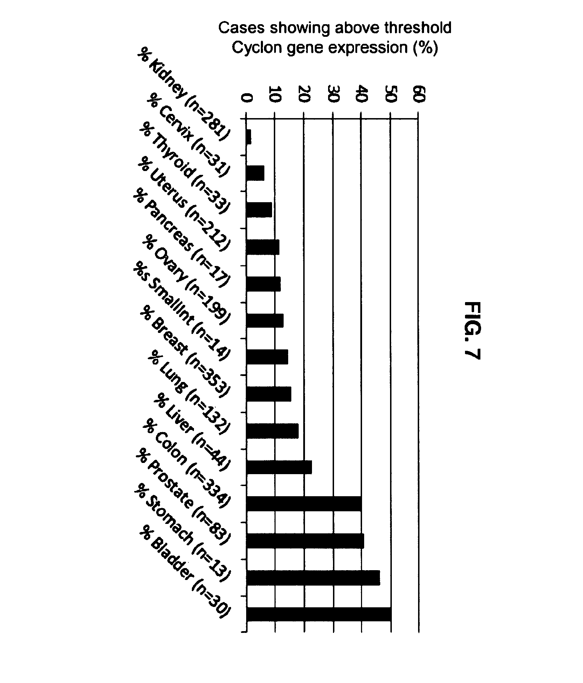

**Figure 7****:** Histogram presenting the percentage of patients showing a Cyclon gene expression above the threshold of expression defined by M+2*sd (M= mean value of expression in a set of 112 normal adult somatic tissues; sd = standard deviation characterizing the distribution of the same values.

### EXAMPLES

Cyclon has been identified as a candidate biomarker for aggressive non Hodgkin lymphoma using an integrative "omics" strategy based on the constitution of a cancer reference proteome followed by a search for "off-context" expression (i.e. aberrant tissue/cell type). The potential of Cyclon to represent a lymphoma biomarker was further confirmed by Cyclon increased gene expression in aggressive lymphoma (Fig. 1) using large-scale lymphoma public transcriptomic data.

Using clinical data associated with public transcriptomic studies (Lenz et al., 2008) we pointed out that Cyclon as a high potential prognosis factor for aggressive non Hodgkin lymphoma that is specific for patients treated by a combination of CD20 agonist (Rituximab) and CHOP chemotherapy (R-CHOP) in Diffuse Large B Cell lymphoma subtypes (Fig. 2).

Using gene silencing experiments in aggressive lymphoma model cell lines (Raji, B593, SUDHL4), we demonstrated that a reduction in Cyclon protein level (sh Cyclon) confers an increased sensitivity of cancer cells to CD20 agonist (Rituximab) treatment both through the direct apoptosis (Fig. 3, left panel) and the complement dependent cytotoxicity (CDC) (Fig. 3, right panel) mechanism compared to control cell lines (sh Ctrl) .

We tested this *in vivo* by subcutaneously injecting control and silenced for Cyclon expression Raji cells. Cyclon knockdown had no obvious impact on the rate of tumour growth *in vivo*. However, upon administration of once daily CD20 agonist (Rituximab) injections, Cyclon-depleted tumours rapidly regressed (by day 4 of treatment) while control tumours continued to grow although these also eventually regressed but with considerably delayed kinetics (day 14 of Rituximab therapy) compared to Cyclon knockdown tumours (Fig. 4).

To further investigate how we could modulate Cyclon expression, we took once gain advantage of transcriptomic gene expression data and identified two druggable pathways, which could reduce Cyclon expression and therefore cause an increased cell susceptibility to Rituximab. We experimentally confirmed that an activation of CD40 pathway can lead to an augmentation of Cyclon mRNA and protein (Fig. 5), suggesting that an antagonist of this signalling pathway could have the opposite effect and therefore be beneficial in a combined therapy involving CD20 agonists.

Another option for combined therapy could involve Imatinib (Glivec) that has been shown to reduce Cyclon expression levels (Fig. 6)

Taken together, these results show that Cyclon is over-expressed in aggressive non-Hodgkin's lymphoma, that it has a prognostic impact for Diffuse Large B cell patients, specifically associated with patients receiving a combined chemotherapy and immunotherapy with CD20 agonist (Rituximab). We further demonstrate that reducing Cyclon expression levels sensitize lymphoma cells to CD20 agonist *in cellulo* and *in vivo* and identify two distinct ways of reducing Cyclon levels, namely CD40 antagonists and Imatinib that could improve patient outcome used in combination with CD20 agonist-based therapy.

Investigating Cyclon gene expression among several cancer types also suggest overexpression of Cyclon can be used to discriminate between non-cancer and cancer cells in others tissues (Fig. 7), namely colon, prostate, stomach and bladder where more than 40% of cancer patients present an above threshold Cyclon gene expression.

### MATERIAL AND METHODS

### Cyclon mRNA detection methods Quantitative PCR

Total RNA was extracted from the cell lines, tissues or sorted B cells by TRIzol reagent (Invitrogen) and quantified by NanoDrop (Thermo Fisher Scientific) before being reverse transcribed using the SuperScript® III First-Strand Synthesis SuperMix for qRT-PCR (Invitrogen), according to manufacturer's instructions. Q-PCR was performed using SYBR® Green PCR master mix (Applied Biosystems) and primers shown in **Table 1**, according to the manufacturer's instructions. The *ACTIN* gene was used as a control gene for normalization of gene expression data. Q-PCR was performed on a MX3000P (Stratagene) machine.

**Table 1**

| **Sh Cloning Duplex Name** | **Oligo Name** | **Sequence 5' to 3'** |
|---|---|---|
| shCy2 | Cyclonsh2F | CGTGTACCGG TCCGTCCAAG AAGTTGAATA AAGCTCGAGC TTTATTCAAC TTCTTGGACG TTTTTGAATT CCGTCGT |
| | Cyclon sh2R | ACGACGGAAT TCAAAAACGT CCAAGAAGTT GAATAAAGCT CGAGCTTTAT TCAACTTCTT GGACGGACCG GTACACG |
| shCy3 | Cyclonsh3F | CGTGTACCGG TCCCCAGATG CTTCAGGACA AGCCTCGAGG CTTGTCCTGA AGCATCTGGG TTTTTGAATT CCGTCGT |
| | Cyclonsh3R | ACGACGGAAT TCAAAAACCC AGATGCTTCA GGACAAGCCT CGAGGCTTGT CCTGAAGCAT CTGGGGACCG GTACACG |
| shCtrl | CtrlshF | CGTGTACCGG TCCCTAAGGT TAAGTCGCCC TCGCTCGAGC GAGGGCGACT TAACCTTAGG TTTTTGAATT CCGTCGT |
| | CtrlshR | ACGACGGAAT TCAAAAACCT AAGGTTAAGT CGCCCTCGCT CGAGCGAGGG CGACTTAACC TTAGGGACCG GTACACG |

| **q-PCR** | **Oligo Name** | **Sequence 5' to 3'** |
|---|---|---|
| | CyclonF | AGCGAAAAGG TTCTTCATCC |
| | CyclonR | TTCCTCTCCT GTCGTTCCTT |

### Expression microarrays

RNA was amplified using the kit GeneChip IVT express (Affymetrix) and hybridized to Affymetrix HG U133 Plus 2.0 microarrays according to manufacturer's instructions (Affymetrix). Fluorescence intensities were quantified using the GCOS 1.2 software. Gene expression data were normalized with MAS5 algorithm using the global scaling method and analyzed using GeneSpring software package (Agilent). Cyclon expression values s **can be retrieved** through the probeset #203119_at.

### Complementary Cyclon detection methods (protein)

### Development of an anti-CYCLON antibody

Anti-CYCLON antibody was obtained after a 28 days rabbit immunization with both C and N-terminus CYCLON peptides. Antibodies were then affinity purified against the C-terminus peptides from immune serum (Speed Rabbit program, Eurogentec).

### Immunoblot

Proteins extracts were resolved by SDS-PAGE and transferred onto a nitrocellulose membrane. After blocking in PBS 8% skimmed milk 0.1% Tween 20, the membranes were incubated with primary antibody (CYCLON, 0.2 µg/ml in PBS 3% skimmed milk 0.1% Tween 20), washed and incubated with anti rabbit IgG-HRP (Thermo Fisher Scientific) before incubation in ECL **SuperSignal West Pico Chemiluminescent Substrate** (Thermo Fisher Scientific). Images were captured using a ChemiDoc XRS+ imager system (Biorad).

### Immmunohistochemistry

Lymph node and spleen paraffined tissue section is stained using Histostain-Plus bulk kit (Invitrogen, Carlsbad, CA, USA) according to manufacturer's instructions. Briefly, tissue sections were deparaffined by 3 successive washes using toluene and then ethanol. After peroxydase quench and serum blocking, slides were incubated with 1 µg/ml anti-Cyclon i.e. 1/100 overnight 4°C, washed and incubated for 10 min with an anti-rabbit IgG biotinylated secondary, then with peroxydase conjugated to streptavidine (HRP-SA) antibody. DAB chromogen is then use to reveal staining. Cell nuclei were counterstained using haematoxylin.

### Cyclon gene silencing

### Gene silencing

Non-targeting or CYCLON targeting short hairpin (sh) RNA sequences were designed using the DSIR algorithm (http://biodev.cea.fr/DSIR/DSIR.html). Sequences, provided in **Table1**, were cloned into the pLKO-1 lentiviral vector (Addgene) and packaged as described(Levy et al., 2010) or purchased from Santa Cruz. Cells were transduced with lentiviral particles at a MOI of 10. Stable cell lines were established under puromycin selection (1 µg/ml).

### Evaluation of Rituximab sensitivity

Cells were treated with 1 to 10 µg/ml Rituximab (Roche) or isotype control (Herceptin, Roche) for 24 h for direct apoptosis assays and for 1 h in presence of 20% human serum as a source of complement for CDC assays. Cell viability was evaluated by AnnexinV/IP (Beckman Coulter) or IP staining and flow cytometry analysis (LSIIR, BD). Specific cell death was calculated as (% of cell death with Rituximab - % cell death with Herceptin)/(1- % cell death with Herceptin), n=8 for each cell line.

### Xenotransplantation assay

3.10⁶ Raji cells were injected subcutaneously into the flank of 6 to 8 week-old nude mice as described. Four mice were injected, each of them with shCtrl and shCyclon Raji cells into the right and left flanks respectively. When tumours were palpable (~0.5 mm³), mice were given daily intraperitoneal injections of 200 µg Rituximab. Tumour volume was measured every 2 days using the formula l²xLx0.52 (n=4 for each group).

### REFERENCES

Basso, K., A.A. Margolin, G. Stolovitzky, U. Klein, R. Dalla-Favera, and A. Califano. 2005. Reverse engineering of regulatory networks in human B cells. Nat Genet. 37:382-90.
Bompas E, Velay B, Blay JY., Leuk Lymphoma. 2004 Nov;45(11):2353-4
Breccia M, Martelli M, Cannella L, Russo E, Finolezzi E, Stefanizzi C, Levi A, Frustaci A, Alimena G., Leuk Res. 2008 Feb;32(2):353-5. Epub 2007 Sep 7.
Cheson, B.D., and J.P. Leonard. 2008. Monoclonal antibody therapy for B-cell non-Hodgkin's lymphoma. N Engl J Med. 359:613-26.
Hoshino, A., and H. Fujii. 2007. Redundant promoter elements mediate IL-3-induced expression of a novel cytokine-inducible gene, cyclon. FEBS Lett. 581:975-80.
Lenz, G., G. Wright, S.S. Dave, W. Xiao, J. Powell, H. Zhao, W. Xu, B. Tan, N. Goldschmidt, J. Iqbal, J. Vose, M. Bast, K. Fu, D.D. Weisenburger, T.C. Greiner, J.O. Armitage, A. Kyle, L. May, R.D. Gascoyne, J.M. Connors, G. Troen, H. Holte, S. Kvaloy, D. Dierickx, G. Verhoef, J. Delabie, E.B. Smeland, P. Jares, A. Martinez, A. Lopez-Guillermo, E. Montserrat, E. Campo, R.M. Braziel, T.P. Miller, L.M. Rimsza, J.R. Cook, B. Pohlman, J. Sweetenham, R.R. Tubbs, R.I. Fisher, E. Hartmann, A. Rosenwald, G. Ott, H.K. Muller-Hermelink, D. Wrench, T.A. Lister, E.S. Jaffe, W.H. Wilson, W.C. Chan, and L.M. Staudt. 2008. Stromal gene signatures in large-B-cell lymphomas. N Engl J Med. 359:2313-23.
Orciuolo E, Fazzi R, Galimberti S, Testi C, Azzara' A, Carulli G, Petrini M., Leuk Res. 2006 Mar;30(3):349-53. Epub 2005 Sep 21
Robak, T., and E. Robak. 2011. New anti-CD20 monoclonal antibodies for the treatment of B-cell lymphoid malignancies. BioDrugs. 25:13-25.
Saint Fleur, S., A. Hoshino, K. Kondo, T. Egawa, and H. Fujii. 2009. Regulation of Fas-mediated immune homeostasis by an activation-induced protein, Cyclon. Blood. 114:1355-65.

## Claims

1. A method for the identification of the presence or absence of cancer cells in a biological sample comprising cells obtained from a patient, said method comprising measuring the level of expression of the gene Cyclon in said cells in the biological sample and comparing the measured level of expression with a standard level of expression in a non-cancer cell, wherein a level of expression higher than the standard level of expression is an indication of the presence of a cancer cell.

2. The method of claim 1, wherein the cancer cells are cells which growth is susceptible to be controlled by a CD20 agonist

3. The method of claims 1 or 2, wherein the cancer cells are selected among colon, prostate, stomach and bladder cancer suspicion.

4. A method for the identification of the susceptibility or resistance to treatment with a CD20 agonists of cancer cells in a biological sample comprising cancer cells obtained from a patient, said method comprising measuring the level of expression of the gene Cyclon in said cancer cells in the biological sample and comparing the measured level of expression with a standard level of expression in a non-cancer cell, wherein a level of expression higher than the standard level of expression is an indication of a resistance to treatment with a CD20 agonists.

5. A method for the identification of a patient having a cancer susceptible to be treated with a CD20 agonist, comprising the step of measuring the level of expression of the gene Cyclon in cancer cells in a biological sample previously obtained from said patient, and comparing the measured level of expression with a standard level of expression in a non-cancer cell, wherein a level of expression lower or about the standard level of expression is an indication of a susceptibility to a treatment with a CD20 agonists.

6. The method of one of claims 4 or 5, wherein the cancer cells are selected among colon, prostate, stomach and bladder cancer.

7. A kit for use in the methods of any one of claims 1 to 6, said kit comprising means for determining the level of expression of Cyclon in cells in a biological sample

8. A CD20 agonist for its use in the treatment of a cancer susceptible to be treated with CD20 agonists, wherein the treatment comprises identifying patients comprising cancer cells susceptible to a treatment with CD20 agonists with the method of one of claims 4 to 6 and administering the CD20 agonist to said susceptible patients.

9. The CD20 agonist for its use in therapy according to claim 8, wherein it is selected among the group consisting of anti-CD20 monoclonal antibodies.

10. A combination product comprising a CD20 agonist and a Cyclon inhibitor, for their use combined or separately, simultaneously of in sequence, in the treatment of cancers susceptible to be treated with CD20 agonists.

11. The combination product of claim 10, wherein the Cyclon inhibitor is an inhibitor of the expression of Cyclon, such as Imatinib.

12. The combination product of claim 11, wherein the Cyclon inhibitor is an inhibitor of the expression of Cyclon selected among the group consisting of CD40 antagonists, such as pathway inhibitor (PG102)

13. The combination product of claim 12, wherein the Cyclon inhibitor is a CD40 antagonist selected among the group consisting of anti-CD40 monoclonal or polyclonal antibodies, such as HCD1222

14. The combination product of one of claims 10 to 13, wherein the CD20 agonist is selected among anti-CD20 monoclonal antibodies, anti-CD20 polyclonal antibodies and antibodies fragments.

15. A method for the identification of compounds susceptible to inhibit the expression of Cyclon in a cancer cell overexpressing Cyclon, said method comprising contacting a sample comprising said cancer cells overexpressing Cyclon with one or more compounds to be tested, measuring the expression level of Cyclon, and comparing the measured expression level with the expression level of the cancer cell overexpressing Cyclon, and selecting the compounds inducing a decrease in said Cyclon expression level.
